# EUROPÄISCHE PATENTANMELDUNG

(11) **EP 1 281 387 A1**
(43) Veröffentlichungstag der Anmeldung: **05.02.2003**
(21) Anmeldenummer: 02090265.6
(22) Anmeldetag: 18.07.2002
(51) Int. Cl.: A61K 7/32, A61K 7/48

(54) **Anti-Transpirant-Kosmetikum auf pflanzlicher Basis**

(30) Priorität: 01.08.2001 DE 10137730
(71) Anmelder: Coty B.V., 2031 CC Haarlem (NL)
(72) Erfinder: Golz-Berner, Karin, MC-9800 Monaco (MC); Zastrow, Leonard, MC-98000 Monaco (MC); Fajon, Virginie, 06200 Nice (FR)
(74) Vertreter: Walter, Wolf-Jürgen

(57) **Zusammenfassung**

Die Erfindung betrifft ein Kosmetikum mit Anti-Schweiß-Wirkung, das für den Einsatz am ganzen Körper geeignet ist und dessen Wirkstoffe im wesentlichen vollständig auf pflanzlicher Basis gewonnen werden. Das Anti-Transpirant-Kosmetikum enthält 0,01 bis 5 Gew-% eines Gemisches des Extraktes der oberirdischen Pflanzenteile von Equisetum arvense und des etherischen Öls von Salvia officinalis; 0,01 bis 5 Gew-% eines Gemisches der Extrakte von Hamamelis virginia und Quercus infectoria; 0,5 bis 15 Gew-% pulverisiertes Bambusholz (Bambuspuder) mit einer Teilchengröße gleich oder kleiner als 15 µm; bis 100 Gew-% kosmetische Hilfs-, Träger- oder weitere Wirkstoffe oder Gemische davon.

## Beschreibung

Die Erfindung betrifft ein Kosmetikum mit Anti-Schweiß-Wirkung, das für den Einsatz am ganzen Körper geeignet ist und dessen Wirkstoffe im wesentlichen vollständig auf pflanzlicher Basis gewonnen werden.

Kosmetika mit desodorierender Wirkung werden eingesetzt, um die beim Schwitzen des menschlichen Körpers entstehenden Geruchsstoffe zumindest zeitweise zu überdecken oder zu binden. Sie werden meist als Lösungen, Puder oder Stifte in den Handel gebracht.

Aus der EP 1002521 ist ein Kosmetikum mit Salbei-Extrakten bekannt. Die DE 199 62 881 beschreibt eine Anti-Transpirant-Zusammensetzung, bestehend aus eine Kombination eines schweißhemmenden Wirkstoffes, wie Salze von Al, Zr oder Zn, mit einem teilchenförmigen wasserlöslichen Polysaccharid und einem Wachs. Aus der DE 4304284 ist ein deodorierendes Mittel auf basis von Lavendelöl und Schachtelhalm-Extrakt bekannt.

Der Erfindung liegt die Aufgabe zugrunde, ein Kosmetikum mit pflanzlichen Wirkstoffen bereitzustellen, das den vom Körper abgegebenen Schweiß aufnimmt und dabei Geruchsstoffe zeitweise nicht zur Geltung kommen läßt.

Erfindungsgemäß besteht das Anti-Transpirant-Kosmetikum aus einem Gemisch von
0,01 bis 5 Gew-% eines Gemisches des Extraktes der oberirdischen Pflanzenteile von Equisetum arvense und des etherischen Öls von Salvia officinalis;
0,01 bis 5 Gew-% eines Gemisches der Extrakte von Hamamelis virginia und Quercus infectoria;
0,5 bis 15 Gew-% pulverisiertes Bambusholz (Bambuspuder) mit einer Teilchengröße gleich oder kleiner als 15 µm; bis 100 Gew-% kosmetische Hilfs-, Träger- oder weitere Wirkstoffe oder Gemische davon, wobei die Gew-% auf das Gesamtgewicht des Kosmetikums bezogen sind.

Der Extrakt von Equisetum arvense wird mit Propylenglycol gewonnen bei Temperaturen von 20-40 °C. Das Verhältnis von Equisetum und Salvia kann im Bereich von 20:80 bis 80:20 liegen. Das Gemisch liegt vorzugsweise in Form einer wäßrigen Lösung in Propylenglycol vor.

Vorteilhaft ist der Zusatz von 0,01 bis 2 Gew-% eines Gemisches von Equisetum arvense und Salvia officinalis, wobei Equisetum auch als gesonderter Bestandteil hinzugesetzt werden kann.

Der Extrakt von Hamamelis Virginia und Quercus infectoria ist ein Extrakt mit Propylenglycol, das aus den oberirdischen Pflanzenteilen bei Temperaturen von 20-40 °C gewonnen wird. Das Verhältnis beider Bestandteile kann im Bereich von 5:95 bis 95:5 liegen. Das Gemisch liegt vorzugsweise in Form einer wäßrigen Lösung in Propylenglycol vor. Es können Zusätze von Aminosäuren wie Glycin, Arginin, Leucin und Gemische davon enthalten sein.

Es können weiterhin 0,01 bis 2 Gew-% eines Wirkstoffes enthalten sein, der ausgewählt ist aus der Gruppe, bestehend aus den etherischen ölen von Eukalyptus, Lemon (Zitrone), Myrrhe, Sandel und Gemischen davon.

Der eingesetzte Bambuspuder besteht vorzugsweise aus dem pulverisierten Mark von Bambusa arundinaceae mit einer bevorzugten mittleren Teilchengröße von etwa 5 µm, wobei etwa 60 % der Teilchen im Bereich von 2-6 µm liegen. Diese spezielle Bambusart wächst in einigen indischen Bergwäldern und eignet sich besonders für die Sebumabsorption und die Texturierung von kosmetischen Produkten. Der Anteil des Bambuspuders liegt vorteilhaft im Bereich von 4-12 Gew-%. Ein besonders bevorzugtes Produkt ist Greensil von Greentech, St. Beauzire, Frankreich.

Als weiteren Wirkstoff kann das Präparat vorteilhaft üblichen Kaolin enthalten oder einen modifizierten Kaolin gemäß WO96/17588, der mit sphärischen TiO₂- oder SiO₂-Teilchen mit einer Teilchengröße <5 µm modifiziert ist, wobei die sphärischen Teilchen einen Anteil an der Kaolinmischung von 0,5 bis 10 Gew-% haben. Das Präparat erhält dadurch ein sehr weiches Hautgefühl und eine zusätzliche Wirksamkeit mit gewisser Entzündungswidrigkeit.

Der modifizierte Kaolin kann einen Anteil von 0,1 bis 17 Gew-% haben, bezogen auf die Gesamtmenge des Kosmetikums.

In einer Ausführungsform der Erfindung kann auch ein Puder aus Methylmethacrylat/Ethylenglycolbismethacrylat-Copolymer zugesetzt werden, der eine durchschnittliche Teilchengröße von etwa 8 µm hat und in Form von makroporösen Kügelchen vorliegt. Der Anteil des PMMA-Puders kann im Bereich von 0,5 bis 10 Gew- % liegen.

Das erfindungsgemäße Kosmetikum kann weiterhin kosmetische Hilfs- und Trägerstoffe enthalten, wie sie üblicherweise in solchen Zubereitungen verwendet werden, z.B. Wasser, Konservierungsmittel, Vitamine, Farbstoffe, Pigmente, Radikalfänger, Verdickungsmittel, weichmachende Substanzen, Duftstoffe, Alkohole, Polyole wie Glycerin und Propylenglycol und Butylenglycol, Ester oder Ether, Elektrolyte, polare und unpolare öle, Polymere, Copolymere, Emulgatoren, Wachse, Gele, Stabilisatoren, Amine wie Triethanolamin oder Gemische davon. Der Zusatz von ölen sollte im Bereich von <5 Gew-% liegen.

Weitere Zusatz- bzw. Wirkstoffe in den kosmetischen Zusammensetzungen können sein Vitamine, z.B. Vitamin A oder Vitamin A-Derivate; organische Lichtschutzmittel wie z.B. Octylmethoxycinnamate; Methyl gluceth 10 oder Methyl gluceth 20.

In einer weiteren Ausführungsform kann das Kosmetikum als Wirkstoff 0,01 bis 2 Gew-% Zinkricinoleate enthalten, das auch in solubilisierter Form zusammen mit Propylenglycol, Triethanolamin und Milchsäure eingebracht werden kann.

Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken sowie Glimmer-Titanoxid-organischer Farbstoff.

Als Ester oder Ether sind zum Beispiel geeignet (INCI-Namen): Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Butyl ether, Dicaprylyl ether, PPG1-PEG9 Lauroyl glycol ether, PPG15 Stearyl ether, PPG14 Butyl ether, PEG20 Stearate, PEG100 Stearate, Fomblin HC25.

In geringen Mengen eingesetzte kosmetische öle können pflanzliche Öle sein, wie Calendulaöl, Jojobaöl, Avocadoöl, Macadamianußöl, Rizinusöl, Weizenkeimöl, Traubenkernöl, Kukuinuß-öl, Distelöl, Nachtkerzenöl, Safloröl oder ein Gemisch mehrerer davon. Es können auch Mineralöle verwendet werden.

Der Zusatz von kosmetischen Gelen kann ebenfalls erfolgen. Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit. Bevorzugt sind Gele auf pflanzlicher Basis.

Es ist weiterhin vorteilhaft, den erfindungsgemäßen Zusamensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Bevorzugt als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch eingesetzt werden können.

Besonders bevorzugt als anorganische Pigmente sind agglomerierte Substrate von TiO₂ und/oder ZnO gemäß WO99/06012, die einen Gehalt an sphärischen und porösen SiO₂-Teilchen aufweisen, wobei die SiO₂-Teilchen eine Teilchengröße im Bereich von 0,05 µm bis 1,5 µm haben, und neben den SiO₂-Teilchen andere anorganische teilchenförmige Stoffe mit sphärischer Struktur vorliegen, wobei die sphärischen SiO₂-Teilchen mit den anderen anorganischen Stoffen definierte Agglomerate mit einer Teilchengröße im Bereich von 0,06 µm bis 5 µm bilden.

Das erfindungsgemäße Kosmetikum kann in Form einer Creme, eines Puders, eines Make-ups, einer Grundierung (foundation), eines sprühbaren Puders vorliegen. Emulsionen dieses Kosmetikums enthalten keinen Filmbildner und sollten allgemein sehr trocken sein.

Das Kosmetikum ist in starkem Maße schweißbindend und stellt in dieser Art eine bemerkenswerte Klasse von Kosmetika dar, da hiermit in erster Linie keine direkte Geruchsunterdrückung beabsichtigt ist, sondern tatsächlich eine starke Schweißaufnahme und -bindung allein durch pflanzliche Wirkstoffe. Damit werden Hautirritationen weitgehend vermieden.

Die Erfindung soll nachstehend durch Beispiele näher erläutert werden. Alle Angaben erfolgen in Gewichtsprozent, sofern nichts anderes angegeben ist.

### Beispiel 1 Körper- und Gesichtscreme

| **Phase A** | |
|---|---|
| Glyceryl Stearate | 5 |
| PEG100 Stearate | 1 |
| Vaseline | 1,5 |
| Paraffin | 0,5 |

| **Phase B** | |
|---|---|
| Wasser | ad 100 |
| Glycerine | 5 |
| Bambuspuder (Bambusa arundinaceae) | 10 |

| **Phase C** | |
|---|---|
| Equisetum arvense Extrakt | 0,01 |
| Hamamelis virginia und | |
| Quercus infectoria Extrakt | 3,2 |
| Eucalyptus Oil | 0,2 |
| Gemisch von Equisetum arvense | |
| und Salvia officinalis | 0,3 |
| Konservierungsmittel | 0,2 |

Die Phasen A und B werden separat durch Vermischen der Ingredienzen hergestellt und auf eine Temperatur von 70-80 °C erhitzt. Unter Rühren werden beide Phase zusammengeführt, mit einem Homogenisator homogenisiert und auf etwa 40 °C abgekühlt. Die Phase C wird nach separater Herstellung durch Vermischen der Einzelkomponenten bei 30-35 °C unter Rühren dem Homogenisat zugesetzt. In einer Variante dieses Beispiels wurde der Phase C 0,5 % Zinkricinoleate zugesetzt.

### Beispiel 2 Hand- und Fußcreme

| **Phase A** | |
|---|---|
| Cetyl Alcohol | 1 |
| Cetearyl Alcohol | 1,5 |
| PEG20 Stearate | 2,5 |
| Cetearyl Octanoate | 1 |

| **Phase B** | |
|---|---|
| Wasser | ad 100 |
| Propylenglycol | 2 |
| Glycerine | 3 |
| Bambuspuder | 5 |

| **Phase C** | |
|---|---|
| Zinkricinoleate | 0,02 |
| Equisetum arvense Extrakt | 3,5 |
| Hamamelis virginia und Quercus infectoria Extrakt | 2,0 |
| Lemon oil | 0,5 |
| Gemisch von Equisetum arvense und Salvia officinalis | 0,3 |
| Konservierungsmittel | 0,2 |

Es wurde wie im Beispiel 1 gearbeitet.

### Beispiel 3 Anti-Transpirant-Puder

| | |
|---|---|
| Kaolin (modifiziert gemäß WO96/7588 | 15 |
| Talcum | ad 100 |
| Zinkricinoleate | 2 |
| Equisetum arvense Extrakt | 5 |
| Hamamelis virginia und Quercus infectoria Extrakt | 5 |
| Sandal Oil | 1 |
| Bambuspuder | 10 |
| Gemisch von Equisetum arvense und Salvia officinalis | 2 |

Die Bestandteile werden in der angegebenen Reihenfolge miteinander vermischt.

### Beispiel 4 (Vergleichsbeispiel 1)

Es wurde ein Studie durchgeführt, bei der von 15 Probanden (männlich und weiblich) im Alter von 14-19 Jahren der transepidermale Wasserverlust (TEWL) von drei Feldern auf den Schulterblättern und am Brustbein gemessen wurde.
Die Versuche wurden wie folgt durchgeführt.
A) ohne Behandlung
B) Creme gemäß Beispiel 1

Power Trail auf dem Ergometer 0 bis 1500 Watt für max. 60 Minuten; Meßsonden TEWL- und EKG-fixiert; bioklimatisierter Raum; erlaubter Puls auf 160 Schläge pro Minute begrenzt. Die Creme wurde 20 Minuten vor Testbeginn aufgetragen.

| Minuten / Watt | Wasserverlust (WE) in g/m² | |
|---|---|---|
| | A | B |
| 5/250 | 21 | 18 |
| 10/250 | 45 | 21 |
| 15/250 | 63 | 32 |
| 20/500 | 82 | 38 |
| 25/500 | 94 | 48 |
| 30/750 | 105 | 75 |
| 40/1000 | 112 | 91 |
| 50/1250 | 123 | 97 |
| 60/1500 | 130 | 105 |

### Beispiel 5 (Vergleichsbeispiel 2)

Es wurde mit der gleichen Anzahl Probanden wie im Beispiel 4 ein physiologischer Power Trail durchgeführt. Meßsonden TEWLund EKG-fixiert; bioklimatisierter Raum; erlaubter Puls auf 160 Schläge pro Minute begrenzt.
Der Test begann mit 15 Minuten Power Trail bei gleichbleibender Belastung von 500 W. Danach 5 Minuten Pause mit Trocknung und Applikation der Creme gemäß Beispiel 1. Danach 15 Minuten Power Trail mit 500 W gleichbleibender Belastung.

| Minuten / Watt | Wasserverlust (WE) in g/m² | |
|---|---|---|
| | A | B |
| 5/250 | 28 | 28 |
| 10/500 | 53 | 53 |
| 15/500 | 65 | 66 |
| PAUSE 5' | | |
| 25/500 | 70 | 49 |
| 30/500 | 101 | 56 |
| 35/500 | 116 | 78 |

Die Vergleichsbeispiel 4 und 5 zeigen mit der Creme B eine ganz klare Wirkung in Bezug auf die Herabsetzung des epidermalen Wasserverlustes, die teilweise über 100 % liegt.

### Beispiel 6 (Vergleichsbeispiel 3)

Ein Test bei verschiedenen Gruppen von Hip-Hop-Tänzern mit einem Durchschnittsalter von 16,6 Jahren bei einer Raumtemperatur von 33 °C und einer relativen Luftfeuchtigkeit von 65% zeigte ebenfalls eine deutliche Absenkung des epidermalen Wasserverlustes (g/m²) um 20-40 % bei einer Tanzdauer von 13 bis 30 Minuten gegenüber unbehandelten Tänzern.

## Patentansprüche

1. Anti-Transpirant-Kosmetikum auf pflanzlicher Basis, **dadurch gekennzeichnet, daß** es enthält
0,01 bis 5 Gew-% eines Gemisches des Extraktes der oberirdischen Pflanzenteile von Equisetum arvense und des etherischen Öls von Salvia officinalis;
0,01 bis 5 Gew-% eines Gemisches der Extrakte von Hamamelis virginia und Quercus infectoria;
0,5 bis 15 Gew-% pulverisiertes Bambusholz (Bambuspuder) mit einer Teilchengröße gleich oder kleiner als 15 µm;
bis 100 Gew-% kosmetische Hilfs-, Träger- oder weitere Wirkstoffe oder Gemische davon, wobei die Gew-% auf das Gesamtgewicht des Kosmetikums bezogen sind.

2. Kosmetikum nach Anspruch 1, **dadurch gekennzeichnet, daß** es als weiteren Wirkstoff 0,01 bis 2 Gew-% Zinkricinoleate enthält.

3. Kosmetikum nach Anspruch 1, **dadurch gekennzeichnet, daß** es als weiteren Wirkstoff Kaolin enthält, der mit sphärischen TiO₂- oder SiO₂-Teilchen mit einer Teilchengröße <5 µm modifiziert ist, wobei die sphärischen Teilchen einen Anteil an der Kaolinmischung von 0,5 bis 10 Gew-% haben.

4. Kosmetikum nach Anspruch 1, **dadurch gekennzeichnet, daß** es 0,01 bis 2 Gew-% des Gemisches von Equisetum arvense und Salvia oficinalis enthält.

5. Kosmetikum nach Anspruch 1, **dadurch gekennzeichnet, daß** es zusätzlich 0,01 bis 2 Gew-% Equisetum arvense Extrakt enthält.

6. Kosmetikum nach Anspruch 1, **dadurch gekennzeichnet, daß** es 0,01 bis 2 Gew-% eines Wirkstoffes enthält, ausgewählt aus der Gruppe, bestehend aus den ölen von Eukalyptus, Lemon, Myrrhe, Sandel und Gemischen davon.

7. Verwendung eines Gemisches, bestehend aus
0,01 bis 5 Gew-% eines Gemisches des Extraktes der oberirdischen Pflanzenteile von Equisetum arvense und des etherischen Öls von Salvia officinalis;
0,01 bis 5 Gew-% eines Gemisches der Extrakte von Hamamelis virginia und Quercus infectoria;
0,5 bis 15 Gew-% pulverisiertes Bambusholz (Bambuspuder) mit einer Teilchengröße gleich oder kleiner als 15 µm;
bis 100 Gew-% kosmetische Hilfs-, Träger- oder weitere Wirkstoffe oder Gemische davon, zur Verringerung des transepidermalen Wasserverlustes.
